# EUROPEAN PATENT APPLICATION

(11) **EP 4 400 488 A1**
(43) Date of publication of application: **17.07.2024**
(21) Application number: 22869683.7
(22) Date of filing: 12.07.2022
(51) Int. Cl.: C07C 1/12, C07C 9/04, C07C 31/04, C22B 5/02, B01D 53/047, B01D 53/62

(54) **GAS SEPARATION METHOD**

(30) Priority: 16.09.2021 JP 2021151551
(71) Applicant: JFE Steel Corporation, Tokyo 100-0011 (JP)
(72) Inventor: SHIGAKI Nobuyuki, Tokyo 100-0011 (JP); YOSHIKAWA Kohei, Tokyo 100-0011 (JP); NISHIKAWA Yuta, Tokyo 100-0011 (JP); TAKAHASHI Koichi, Tokyo 100-0011 (JP); KASHIHARA Yusuke, Tokyo 100-0011 (JP)
(74) Representative: Grünecker Patent- und Rechtsanwälte PartG mbB
(86) International application number: PCT/JP2022/027480
(87) International publication number: WO 2023/042535

(57) **Abstract**

Provided is a gas separation method that can separate and supply a suitable gas from a source gas to each of a plurality of gas utilizing apparatuses by a pressure swing adsorption method. The method includes a target gas component adsorption process in which the source gas is introduced into an adsorbent vessel filled with an adsorbent and the target gas component is adsorbed on the adsorbent, a target gas component desorption process in which the target gas component is desorbed to discharge a desorbed gas containing the target gas component from the adsorbent vessel, and a desorbed gas supply process in which the desorbed gas is supplied to two or more different gas utilizing apparatuses, the target gas component desorption process having a desorbed gas utilizing apparatus switching process for switching a supply destination of the desorbed gas in the desorbed gas supply process among the apparatuses.

## Description

### TECHNICAL FIELD

This disclosure relates to a gas separation method.

### BACKGROUND

Conventionally, the pressure swing adsorption (PSA) method has been used as a method for separating predetermined gas components contained in a source gas (see, for example, JPH06-144818A (PTL 1)). The PSA method is a separation method that utilizes the fact that the amount of a gas component adsorbed on an adsorbent depends on the gas species and partial pressure thereof. The PSA method usually includes a process in which a gas component is adsorbed on an adsorbent (adsorption process), a process in which part of a desorbed gas desorbed in another adsorbent vessel is supplied as rinse gas to increase the adsorption ratio of the gas component on the adsorbent (rinse process), and a process in which the adsorbed gas component is desorbed from the adsorbent and recovered (desorption process).

The PSA method described above, which has been applied in various fields, is often used as a method for producing highly concentrated gas by adsorbing a gas component contained in a source gas. The PSA method includes a pressurizing type utilizing a difference between increased pressure and normal pressure and a vacuum type utilizing a difference between normal pressure (or slightly increased pressure) and reduced pressure. The latter type is also called VSA (vacuum swing adsorption) method.

In recent years, the carbon dioxide capture and utilization (CCU) process, which produces useful chemicals such as methanol from carbon dioxide (CO₂) emitted from steelmaking processes, etc., has been studied. Because the reaction process such as CCU can use even a source gas without high purity, the gas separation apparatus can be made inexpensive.

As a gas separation apparatus suitable for CCU, we proposed, in JP2018-114464A (PTL 2), a gas separation method based on the difference in gas desorption time, utilizing the characteristic that the time taken for gas to desorb from the adsorbent varies depending on the adsorbed gas species. This method can efficiently separate CO₂, which has stronger adsorption affinity, from CO and N₂, which have weaker adsorption affinity, among blast furnace gas (containing CO₂, carbon monoxide (CO), and nitrogen (N₂)) emitted from, for example, a steelmaking process. Here, the CO₂ to be separated is precisely a gas (CO₂-rich gas) that also contains some impurity gases such as CO and N₂, but hereinafter referred to as CO₂ for the sake of simplicity of explanation.

### CITATION LIST

### Patent Literature

PTL 1: JPH06-144818A
PTL 2: JP2018-114464A

### SUMMARY

### (Technical Problem)

The simple gas separation method described in PTL 2 can be used to reduce the production cost of CCU chemicals. On the other hand, however, the gas separated by the method described in PTL 2 may be separated as a gas containing many impurities depending on the operation method. This may affect the catalytic performance of the reactor in the CCU process.

Various catalysts are used in the reactor used for CCU, depending on the intended reaction product. The catalytic performance is affected not only by reaction conditions such as temperature and pressure, but also by the composition of the source gas. For example, when a catalyst that is easily deactivated by impurity gas components other than CO₂ is used, high-purity CO₂ is required. On the other hand, because an impurity gas component that does not affect the catalyst performance can be fed as it is to the reactor, high-purity CO₂ is not necessary.

Thus, when the required purity of the source gas CO₂ is different for each catalyst of the reactor, it is necessary to select the appropriate CO₂ separation apparatus depending on CO₂ purity. For example, when high-purity CO₂ is required, a CO₂ separation apparatus that can produce high-purity CO₂, such as in the chemical absorption method, is suitable. When high-purity CO₂ is not required, CO₂ can be separated simply by using, for example, the PSA method described above.

Meanwhile, when different CO₂ separation apparatuses are installed in reactors, respectively, in this manner, various disadvantages arise, such as increased construction costs and increased operating personnel due to the installation of multiple apparatuses. In addition, the production volume of each reactor is constrained by the processing capacity of the CO₂ separation apparatus, making it impossible to flexibly adjust the production volume balance among the reactors.

It could thus be helpful to provide a gas separation method that can separate and supply a suitable gas from a source gas to each of a plurality of gas utilizing apparatuses by using the pressure swing adsorption method.

### (Solution to Problem)

We thus provide the following.
[1] A gas separation method for adsorbing and separating a target gas component from a source gas containing two or more gas components by a pressure swing adsorption method, comprising
   a target gas component adsorption process in which the source gas is introduced into an adsorbent vessel filled with an adsorbent and the target gas component is adsorbed on the adsorbent,
   a target gas component desorption process in which the target gas component adsorbed on the adsorbent in the target gas component adsorption process is desorbed to discharge a desorbed gas containing the target gas component from the adsorbent vessel, and
   a desorbed gas supply process in which the desorbed gas discharged from the adsorbent vessel in the target gas component desorption process is supplied to two or more different gas utilizing apparatuses, wherein
   the target gas component desorption process has a desorbed gas utilizing apparatus switching process in which a supply destination of the desorbed gas in the desorbed gas supply process is switched among the gas utilizing apparatuses.
[2] The gas separation method according to [1], wherein the source gas contains at least carbon dioxide and carbon monoxide.
[3] The gas separation method according to [1] or [2], wherein the source gas is an exhaust gas discharged from a reduction furnace.
[4] The gas separation method according to any one of [1] to [3], wherein in the desorbed gas utilizing apparatus switching process, the supply destination is switched among the gas utilizing apparatuses at boundaries of multiple time periods that are produced by dividing the target gas component desorption process.
[5] The gas separation method according to any one of [1] to [4], further comprising, upstream of the desorbed gas supply process, one or both of a desulfurization process in which sulfur is removed from the source gas or the desorbed gas, and a water gas shift reaction process in which carbon dioxide is generated from the carbon monoxide and water contained in the source gas or the desorbed gas.
[6] The gas separation method according to any one of [1] to [5], wherein the target gas component adsorption process further has an adsorption off-gas supply process in which an adsorption off-gas containing a non-adsorbed gas component not adsorbed on the adsorbent in the target gas component adsorption process is supplied to one of the two or more different gas utilizing apparatuses.
[7] The gas separation method according to [6], wherein the target gas component adsorption process has an adsorption off-gas utilizing apparatus switching process for switching a supply destination of the adsorption off-gas among the gas utilizing apparatuses based on a concentration of the target gas component in the adsorption off-gas.
[8] The gas separation method according to any one of [1] to [7], further comprising, downstream of the target gas component desorption process and upstream of the desorbed gas supply process, a gas mixing process in which the desorbed gases with different concentrations of the target gas component discharged from the adsorbent vessel are mixed.
[9] The gas separation method according to any one of [1] to [8], wherein one of the two or more gas utilizing apparatuses is a methanol synthesis reactor.
[10] The gas separation method according to any one of [1] to [9], wherein one of the two or more gas utilizing apparatuses is a methane synthesis reactor.
[11] The gas separation method according to any one of [1] to [8], wherein one of the two or more gas utilizing apparatuses is a CO₂ liquefaction apparatus.
[12] The gas separation method according to [10] citing [2] or [3], or [11] citing [2] or [3], wherein a product of the gas utilizing apparatuses is supplied as a reducing agent in the reduction furnace.

### (Advantageous Effect)

According to this disclosure, the pressure swing adsorption method can separate and supply a suitable gas from the source gas to each of a plurality of gas utilizing apparatuses.

### BRIEF DESCRIPTION OF THE DRAWINGS

In the accompanying drawings:
FIG. 1 illustrates a flowchart of the gas separation method according to this disclosure; and
FIG. 2 illustrates a flow diagram of the gas separation process in the example.

### DETAILED DESCRIPTION

The following illustrates an embodiment of this disclosure with reference to the drawings. The gas separation method according to this disclosure, which is a gas separation method for adsorbing and separating a target gas component from a source gas containing two or more gas components by a pressure swing adsorption method, includes a target gas component adsorption process in which the source gas is introduced into an adsorbent vessel filled with an adsorbent and the target gas component is adsorbed on the adsorbent, a target gas component desorption process in which the target gas component adsorbed on the adsorbent in the target gas component adsorption process is desorbed to discharge a desorbed gas from the adsorbent vessel, and a desorbed gas supply process in which the desorbed gas discharged from the adsorbent vessel in the target gas component desorption process is supplied to two or more different gas utilizing apparatuses. The target gas component desorption process has a desorbed gas utilizing apparatus switching process in which a supply destination of the desorbed gas in the desorbed gas supply process is switched among the gas utilizing apparatuses.

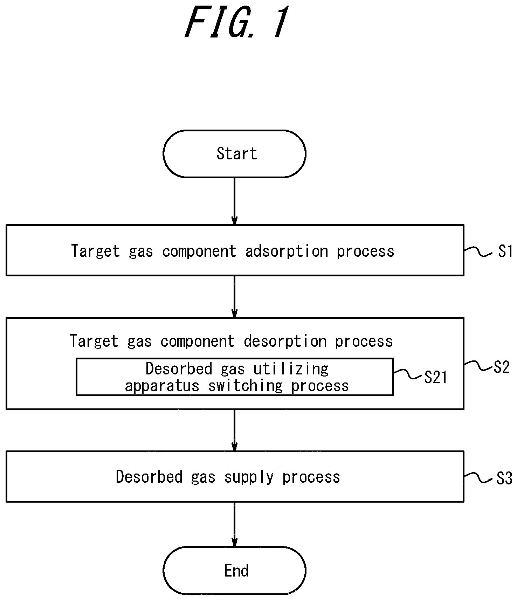

FIG. 1 illustrates a flowchart of the gas separation method according to this disclosure. First, in Step S1, a source gas is introduced into an adsorbent vessel filled with an adsorbent and a target gas component is adsorbed on the adsorbent (target gas component adsorption process).

The source gas is not limited as long as it contains two or more gas components, but it should contain at least CO₂ and CO. While CO₂ and CO can contribute to global warming if released into the air, they can be effectively used as raw materials for synthesizing fuels such as methane and methanol, and as reducing agents in a reduction furnace. CO can also be used as a fuel gas because of its high heat quantity. Therefore, the source gas should contain at least CO₂ and CO.

As such CO₂- and CO-containing source gases, a by-product gas generated at steelworks can be used. As the by-product gas, exhaust gas emitted from a reduction furnace such as a blast furnace and shaft furnace can be used. In particular, blast furnace gas emitted from a blast furnace contains not only CO₂ richly, but also contain CO. When the source gas contains CO, there are concerns about catalyst degradation due to carbon precipitation caused by CO, depending on the catalyst used by the gas utilizing apparatuses supplied in the desorbed gas supply process described below. However, in this disclosure, the CO content of the desorbed gas supplied to such apparatuses can be controlled, so a blast furnace gas can be used as the source gas without problems.

The source gas as described above is introduced into an adsorbent vessel filled with an adsorbent and a target gas component is adsorbed on the adsorbent. The adsorbent filled in the adsorbent vessel can be appropriately selected depending on the target gas component to be adsorbed on the adsorbent. The target gas component is not limited and can be, for example, CO₂ or CO. When the target gas component is CO₂, 13X zeolite or the like can be suitably used as the adsorbent.

Next, in Step S2, the target gas component adsorbed on the adsorbent in Step S 1 is desorbed to discharge a desorbed gas containing the target gas component from the adsorbent vessel (target gas component desorption process). Desorption of the target gas component from the adsorbent and discharge from the adsorbent vessel can be performed by connecting a vacuum pump to the adsorbent vessel.

In this disclosure, using changes over time in the composition of the desorbed gas (concentration of the target gas component), the desorption process is divided into multiple time periods and the desorbed gas is discharged separately for each time period. In detail, for example, 13X zeolite used as an adsorbent for CO₂ has linear adsorption properties with respect to pressure for gas components with weaker adsorption affinity such as N₂ and CO. In contrast, for gas components with stronger adsorption affinity such as CO₂, it has a large adsorption volume and nonlinear adsorption properties with respect to pressure.

When an adsorbent having different adsorption/desorption properties with respect to pressure depending on the gas component is used, there will be differences in the timing of desorption of the gas component in the desorption process. Specifically, a gas component with linear adsorption properties with respect to pressure (e.g., CO, N₂) is desorbed in the depressurization process from a high-pressure state. In contrast, a gas component (e.g., CO₂) with nonlinear adsorption properties with respect to pressure is hardly desorbed under high-pressure conditions and rapidly desorbed when the pressure is reduced to low.

Depending on such difference in the desorption timing of gas component, for example, a desorbed gas in the early stage of the desorption process, which contains many impurity gas components, is fed to a gas utilizing apparatus that tolerate a source gas containing impurities. In contrast, a desorbed gas in the latter stage of the desorption process, which contains few impurity gas components, is fed to another gas utilizing apparatus that requires high-purity source gas. The amount of desorbed gas fed to these gas utilizing apparatuses and the concentration of the target gas component in the desorbed gas can be easily adjusted by changing the length of the time period produced by dividing the desorption process.

In Step S21, a supply destination of the desorbed gas in the desorbed gas supply process in Step S3 described below is switched among the gas utilizing apparatuses (desorbed gas utilizing apparatus switching process). The switching can be performed at the boundaries of multiple time periods that are produced by dividing the desorption process. This allows the desorbed gas to be fed to an appropriate gas utilizing apparatus based on its composition (concentration of target gas component).

Then, in Step S3, the desorbed gas discharged from the adsorbent vessel in Step S2 is fed to two or more different gas utilizing apparatuses (desorbed gas supply process). The desorbed gas is thus fed to an appropriate gas utilizing apparatus based on its composition (concentration of target gas component).

Here, the "gas utilizing apparatus" means an apparatus such as a reactor, reformer, and condenser that chemically or physically transforms the desorbed or adsorbed off-gas to obtain a target substance. The reactor includes a methanol synthesis reactor and methane synthesis reactor. The reformer includes a water gas shift reformer and dry reforming apparatus that reacts methane with CO₂. The condenser includes a CO₂ liquefaction apparatus and gas deep-cooling separation apparatus.

Of the above, one of the two or more different gas utilizing apparatuses is preferably a methanol synthesis reactor. Methanol can be used as a fuel and produced by the reaction of the equation (1) of CO₂ and H₂ or the equation (2) of CO and H₂.

CO₂ + 3H₂ → CH₃OH + H₂O (1)

CO + 2H₂ → CH₃OH (2)

Cu-Zn catalysts commonly used in methanol synthesis are less susceptible to degradation of catalytic performance due to carbon precipitation even when a CO-containing source gas is used. Rather, the source gas containing even a small amount of CO has the advantage of improving the yield of the reaction of converting CO₂ to methanol because the side reaction of converting CO₂ to CO is suppressed. Thus, when the gas utilizing apparatus is a methanol synthesis reactor, even a desorbed gas without a high concentration of CO₂ can be used as a source gas for methanol synthesis. Therefore, a desorbed gas obtained in the middle stage of the desorption process when a relatively high concentration of CO is desorbed in the desorption process can be fed to the methanol synthesis reactor and the concentration control of the desorbed gas in this disclosure can be effectively used.

Further, one of the two or more different gas utilizing apparatuses is preferably a methane synthesis reactor. Methane can be used not only as a fuel, but also as a reducing agent for the reduction of oxides in the reduction furnace. Methane can be produced by the reaction of the equation (3) of CO₂ and H₂ or the equation (4) of CO and H₂.

CO₂ + 4H₂ → CH₄ + 2H₂O (3)

CO + 3H₂ → CH₄ + H₂O (4)

Ni catalysts used in methane synthesis from CO₂ may suffer from degradation of catalytic performance due to carbon precipitation when a CO-containing source gas is used. Therefore, a desorbed gas obtained in the latter stage of the desorption process when a high concentration of CO₂ is desorbed in the desorption process is fed to the methanol synthesis reactor.

Furthermore, one of the two or more different gas utilizing apparatuses is preferably a CO₂ liquefaction apparatus. When the source gas is a mixed gas containing CO₂ and CO and the adsorbent is a zeolite, most of the gas components that are desorbed in the latter stage of the desorption process are CO₂ that has stronger adsorption affinity on the adsorbent, and thus the desorbed gas is discharged as high-purity CO₂ gas. Therefore, the desorbed gas discharged in the latter stage of the desorption process can be fed to the CO₂ liquefaction apparatus for CO₂ storage (CCS) or other purposes.

When the source gas is a gas containing at least CO₂ and CO, or exhaust gas discharged from the reduction furnace, the product of the gas utilizing apparatus can be supplied as a reducing agent in the reduction furnace. The product in the gas utilizing apparatus is not limited to the above methanol and is not limited to any particular substance as long as it has a reducibility to raw materials of reduction furnaces such as iron ore. The physical properties, such as gas or liquid, are not limited, either.

For example, when the gas utilizing apparatus is a methane synthesis reactor, the methane produced has a reducing effect on oxides such as iron ore and thus can be used as a reducing agent in the reduction furnace. In particular, when the source gas for the methane synthesis reactor is CO₂, it is possible to establish a carbon-recycling reduction process in which CO₂ is converted to methane and C is recycled. The methane synthesis reaction is an exothermic reaction, and by using the high-temperature methane produced as it is as a reducing agent in the reduction furnace, the reaction heat of the methane synthesis can also be effectively utilized.

In this way, the PSA method can separate and supply a suitable gas from the source gas to each of a plurality of gas utilizing apparatuses.

This disclosure can further comprise, upstream of the desorbed gas supply process, one or both of a desulfurization process in which sulfur is removed from the source gas or desorbed gas, and a water gas shift reaction process in which CO₂ is generated from CO and water (H₂O) contained in the source gas or desorbed gas.

In detail, when in the gas utilizing apparatus, catalytic performance is prone to be degraded due to sulfur, sulfur contained in the desorbed gas is preferably removed in a desulfurization apparatus before the gas is fed to the gas utilizing apparatus (desulfurization process). Thus, the desulfurization process can be performed or not depending on the performance of the catalyst, etc. used in the gas utilizing apparatus.

When the desulfurization process is performed, it may be performed at any stage upstream of the desorbed gas supply process that supplies a desorbed gas to the gas utilizing apparatus. For example, the desulfurization process can be performed by installing a desulfurization apparatus upstream of any one of the gas utilizing apparatuses. In addition, a desulfurization apparatus can be installed upstream of the PSA apparatus to remove sulfur from the source gas, and then feed the source gas after desulfurization to the PSA apparatus so that all gas utilizing apparatuses are supplied with desulfurized desorbed gas.

The water gas shift reaction process is a process to cause the reaction of CO and H₂O in the equation (5), and the balance between CO and CO₂ concentrations can be adjusted by the reaction ratio.

CO + H₂O → CO₂ + H₂ (5)

For example, when the allowable CO concentration is small or the required CO₂ concentration is high in the gas utilizing apparatus, the requirements of the gas utilizing apparatus can be satisfied by performing the water gas shift reaction process as a pretreatment process of the desorbed gas supply process.

Both the desulfurization process and water gas shift reaction process can be performed as appropriate according to the specifications of the gas utilizing apparatus. Both the desulfurization process and water gas shift reaction process can be performed consecutively upstream of the desorbed gas supply process. In such case, it is preferable to perform the water gas shift reaction process after the desulfurization process. By performing the processes in this order, sulfur in the gas is removed before the gas is fed to the apparatus where the water gas shift reaction takes place. This is more desirable because the catalyst in the apparatus where the water gas shift reaction process is performed will not be in contact with sulfur, which slows the catalyst degradation rate.

In addition, it is preferable to further comprise, downstream of the target gas component desorption process and upstream of the desorbed gas supply process, a gas mixing process in which the desorbed gases with different concentrations of the target gas component discharged from the adsorbent vessel are mixed. In detail, when the desorbed gas discharged during each of the multiple time periods in the desorption process is fed as it is to the gas utilizing apparatus, composition fluctuations of the desorbed gas will directly lead to composition fluctuations of the source gas in the gas utilizing apparatus, which may affect the production efficiency of the gas utilizing apparatus. Therefore, it is preferable to install a tank or other gas mixing apparatus upstream of the gas utilizing apparatus to collect and thoroughly mix the desorbed gas discharged during a given period to equalize the composition of the desorbed gas and reduce the effects of composition fluctuations before supplying the desorbed gas to the gas utilizing apparatus. This can reduce the effect on the production efficiency of the gas utilizing apparatus.

In the desorbed gas supply process, the gas supplied to the gas utilizing apparatus is the desorbed gas obtained in the target gas component desorption process. In this disclosure, in addition, an adsorption off-gas containing a non-adsorbed gas component that was not adsorbed by the adsorbent in the target gas component adsorption process can be fed to the gas utilizing apparatus (adsorption off-gas supply process). In detail, when the gas utilizing apparatus has a large allowable amount of impurity gas (e.g., CO) other than the main reaction gas (e.g., CO₂), gas containing a large amount of impurity gas, such as an adsorption off-gas containing a non-adsorbed gas component in the adsorption process of the PSA apparatus, can also be fed and used in the gas utilizing apparatus.

Here, the "non-adsorbed gas component", which simply means a gas component that was not adsorbed on the adsorbent during the adsorption process, does not mean a gas component without adsorption affinity on the adsorbent. For example, when the source gas contains CO₂, CO, and H₂ and the adsorbent is a zeolite, the adsorption affinity on the adsorbent is higher in order of CO₂, CO, and H₂. Therefore, H₂ makes up the majority of the adsorption off-gas at the early stage of the adsorption process. However, when the adsorption process is continued, CO₂ is no longer adsorbed on the adsorbent due to breakthrough. Such CO₂ that was not adsorbed on the adsorbent due to breakthrough is also included in the "non-adsorbed gas component".

When the adsorption off-gas is fed to the gas utilizing apparatus as described above, a supply destination of the adsorption off-gas is preferably switched among the gas utilizing apparatuses based on a concentration of the target gas component in the adsorption off-gas (adsorption off-gas utilizing apparatus switching process).

In detail, the composition (concentration of target gas component) of the adsorbed off-gas containing a non-adsorbed gas component that was not adsorbed on the adsorbent in the target gas component adsorption process changes with time, as does the adsorbed gas. As described above, for example, when the source gas contains CO₂, CO, and H₂ and the adsorbent is a zeolite, the adsorption off-gas in the early stage of the adsorption process is mostly H₂, and the concentration of CO increases as the adsorption process proceeds. Therefore, an off-gas in the early stage of the adsorption process can be fed to a gas utilizing apparatus that uses H₂ and an off-gas in the middle stage of the adsorption process can be fed to an apparatus that uses CO₂, thus making it possible to feed the appropriate gas to each of the gas utilizing apparatuses depending on the composition of the adsorption off-gas.

### EXAMPLES

Hereinafter, the example of this disclosure will be described, but this disclosure is not limited to it.

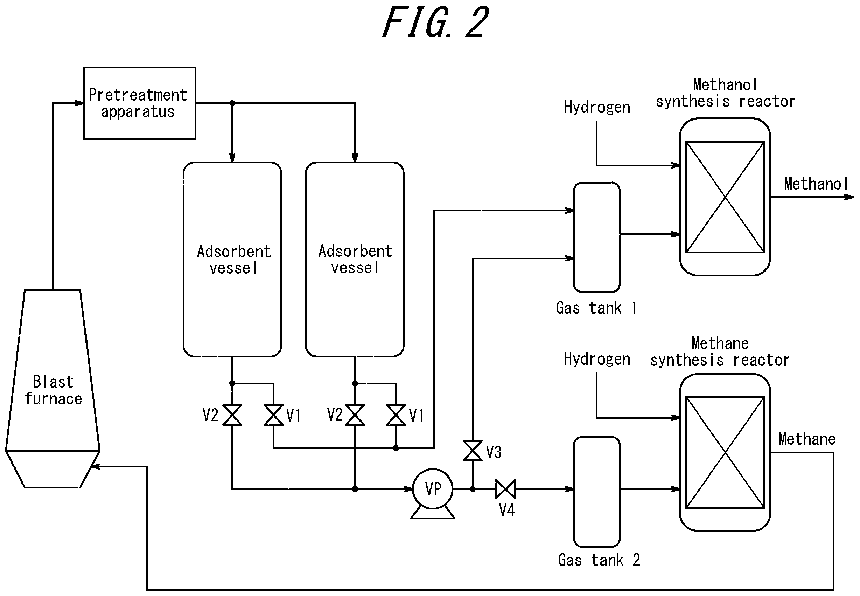

According to the gas separation process flow diagram illustrated in FIG. 2, a blast furnace gas was introduced into a gas separation apparatus and a desorbed gas was fed to the gas utilizing apparatus. Specifically, first, the blast furnace gas discharged from a blast furnace that is a reduction furnace was reformed and cleaned by a pretreatment apparatus. The reformed and cleaned gas was then introduced as a source gas into one of two adsorbent vessels that constitute the gas separation apparatus (PSA apparatus), where a valve V1 in the exhaust line is open and a valve V2 is closed (in the other adsorbent vessel, the valve V1 is closed and V2 is open). CO₂ contained in the blast furnace gas was adsorbed onto an adsorbent (13X zeolite) filled in the adsorbent vessel (target gas component adsorption process). An adsorbed off-gas that was not adsorbed on the adsorbent in the target gas component adsorption process was introduced into a gas tank 1 positioned upstream of a methanol synthesis reactor that uses a Cu-Zn catalyst.

Next, CO₂ adsorbed on the adsorbent was desorbed by a vacuum pump VP, and the desorbed gas containing CO₂ was discharged from the adsorbent vessel (target gas component desorption process). The target gas component desorption process was divided into two time periods according to the changes over time of the composition of the adsorption gas. During the first period of the desorption process, in the adsorbent vessels, the valve V1 was closed, valve V2 was open, a valve V3 was open, and a valve V4 was closed, and an adsorption gas with a relatively low CO₂ concentration was introduced into the gas tank 1 positioned upstream of the methanol synthesis reactor and mixed with the adsorption off-gas (mixing process). Then, during the second period of the desorption process, the valve V3 was closed and valve V4 was open to switch the supply destination of desorbed gas to a methane synthesis reactor, and a desorbed gas with a relatively high CO₂ concentration was introduced into a gas tank 2 positioned upstream of the methane synthesis reactor that uses a Ni catalyst. When the gases introduced into the gas tank 1 and gas tank 2 were sufficiently mixed, respectively, the gas in the gas tank 1 was fed to the methanol synthesis reactor along with H₂ gas to synthesize methanol. Meanwhile, the gas in the gas tank 2 was fed to the methane synthesis reactor along with H₂ gas to synthesize methane. The synthesized methane was fed to the blast furnace that is a reduction furnace as a reducing agent to reduce oxides contained in iron ores.

### INDUSTRIAL APPLICABILITY

The pressure swing adsorption method, which can separate and supply a suitable gas from a source gas to each of a plurality of gas utilizing apparatuses, is useful in steelmaking industry.

## Claims

1. A gas separation method for adsorbing and separating a target gas component from a source gas containing two or more gas components by a pressure swing adsorption method, comprising
a target gas component adsorption process in which the source gas is introduced into an adsorbent vessel filled with an adsorbent and the target gas component is adsorbed on the adsorbent,
a target gas component desorption process in which the target gas component adsorbed on the adsorbent in the target gas component adsorption process is desorbed to discharge a desorbed gas containing the target gas component from the adsorbent vessel, and
a desorbed gas supply process in which the desorbed gas discharged from the adsorbent vessel in the target gas component desorption process is supplied to two or more different gas utilizing apparatuses, wherein
the target gas component desorption process has a desorbed gas utilizing apparatus switching process in which a supply destination of the desorbed gas in the desorbed gas supply process is switched among the gas utilizing apparatuses.

2. The gas separation method according to claim 1, wherein the source gas contains at least carbon dioxide and carbon monoxide.

3. The gas separation method according to claim 1 or 2, wherein the source gas is an exhaust gas discharged from a reduction furnace.

4. The gas separation method according to any one of claims 1 to 3, wherein in the desorbed gas utilizing apparatus switching process, the supply destination is switched among the gas utilizing apparatuses at boundaries of multiple time periods that are produced by dividing the target gas component desorption process.

5. The gas separation method according to any one of claims 1 to 4, further comprising, upstream of the desorbed gas supply process, one or both of a desulfurization process in which sulfur is removed from the source gas or the desorbed gas, and a water gas shift reaction process in which carbon dioxide is generated from the carbon monoxide and water contained in the source gas or the desorbed gas.

6. The gas separation method according to any one of claims 1 to 5, wherein the target gas component adsorption process further has an adsorption off-gas supply process in which an adsorption off-gas containing a non-adsorbed gas component not adsorbed on the adsorbent in the target gas component adsorption process is supplied to one of the two or more different gas utilizing apparatuses.

7. The gas separation method according to claim 6, wherein the target gas component adsorption process has an adsorption off-gas utilizing apparatus switching process for switching a supply destination of the adsorption off-gas among the gas utilizing apparatuses based on a concentration of the target gas component in the adsorption off-gas.

8. The gas separation method according to any one of claims 1 to 7, further comprising, downstream of the target gas component desorption process and upstream of the desorbed gas supply process, a gas mixing process in which the desorbed gases with different concentrations of the target gas component discharged from the adsorbent vessel are mixed.

9. The gas separation method according to any one of claims 1 to 8, wherein one of the two or more gas utilizing apparatuses is a methanol synthesis reactor.

10. The gas separation method according to any one of claims 1 to 9, wherein one of the two or more gas utilizing apparatuses is a methane synthesis reactor.

11. The gas separation method according to any one of claims 1 to 8, wherein one of the two or more gas utilizing apparatuses is a CO₂ liquefaction apparatus.

12. The gas separation method according to claim 10 citing claim 2 or 3, or claim 11 citing claim 2 or 3, wherein a product of the gas utilizing apparatuses is supplied as a reducing agent in the reduction furnace.
